# EUROPÄISCHE PATENTSCHRIFT

(11) **EP 1 872 748 B1**
(45) Veröffentlichungstag und Bekanntmachung des Hinweises auf die Patenterteilung: **23.12.2009**
(21) Anmeldenummer: 07005220.4
(22) Anmeldetag: 14.03.2007
(51) Int. Cl.: A61F 2/44

(54) **Implantat zum Einsetzen zwischen zwei Wirbelkörper der Wirbelsäule**
Implant for inserting between two vertebrae of the spine
Implant destiné à l'intégration entre deux vertèbres de la colonne vertébrale

(30) Priorität: 28.06.2006 DE 102006030124
(43) Veröffentlichungstag der Anmeldung: 02.01.2008
(73) Patentinhaber: M.O.R.E. Medical Solutions GmbH, 18211 Börgerende (DE)
(72) Erfinder: Grandl, Georg, Dr., 18211 Börgerende (DE); Khodadadyan-Klostermann, Cyrus, Dr., 32756 Detmold (DE)
(74) Vertreter: Hentrich, Swen

(56) Entgegenhaltungen:
- EP-A2- 0 950 388
- WO-A-2005/112834
- DE-A1- 19 622 827
- US-A1- 2003 208 272
- US-A1- 2004 167 626
- US-A1- 2005 071 007

## Beschreibung

Die Erfindung betrifft ein Implantat zum Einsetzen zwischen zwei Wirbelkörper der Wirbelsäule als Platzhalter für aus der Wirbelsäule entfernte Bandscheiben und Wirbel oder Wirbelteile, mit zwei Implantatteilen, die in Richtung ihrer koaxialen Längsachsen zur Längenänderung des Implantats gegeneinander verstellbar sind.

Derartige aus der Praxis bekannte Implantate finden Verwendung, wenn bei degenerativen Erkrankungen der Wirbelsäule oder nach deren traumatischer Beschädigung die Wirbelsäule wieder stabilisiert werden muss, damit diese ihre tragende Funktion ausführen kann. Dazu werden die bekannten Implantate in die Lücke zwischen zwei Wirbelkörper eingesetzt, die entstanden ist, indem ein Wirbel oder Wirbelteile mit den benachbarten Bandscheiben entfernt worden ist. Durch die bekannten Implantate wird eine Fusion der benachbarten Wirbelkörper erzielt, durch die die Kraftübertragungskette wieder geschlossen ist. Allerdings ist mit dieser Fusion auch eine Versteifung verbunden.

Ein Implantat gemäß dem Oberbegriff des Anspruchs 1 ist aus dem Dokument US-A-2004/016762 bekannt.

Der Erfindung liegt daher die Aufgabe zugrunde, ein Implantat der eingangs genannten Art so auszubilden, dass bei völligem Erhalt der Distrahierbarkeit der beiden Implantatteile eine Eignung des Implantats als Bandscheibenprothese erreicht wird, um eine Beweglichkeit in den betroffenen Segmenten der Wirbelsäule zu erhalten.

Diese Aufgabe wird nach der Erfindung bei einem Implantat der eingangs genannten Art dadurch gelöst, dass eine an einem der Wirbelkörper befestigbare Lagerplatte mit einem gekrümmten Lagersitz vorgesehen ist, an dem eines der Implantatteile mit einer korrespondierend gekrümmten Lagerfläche anliegt.

Durch diese Gestaltung wird zunächst erreicht, dass zwischen dem einen Implantatteil und dem Wirbelkörper eine Lagerplatte angeordnet ist, die zur Erzielung der gewünschten Beweglichkeit eine Relativbewegung des Implantatteils gegenüber der Lagerplatte ermöglicht, also das Implantatteil nicht selber gegenüber dem Wirbelkörper in direktem Kontakt verstellt werden muss. Die Lagerplatte kann daher dauerhaft und stabil an dem Wirbelkörper befestigt werden und dabei durch das Implantatteil eine großflächige Krafteinleitung mit geringer Flächenpressung ermöglichen, um eine Beschädigung des Wirbelkörpers selber zu vermeiden. Diese in der Wirbelsäule bereitgestellte Beweglichkeit wird durch eine Verstellung des Implantatteils gegenüber dem Lagersitz erzielt, wobei die gewünschten Kippbewegungen durch das Abrollen der einander anliegenden Flächen von Lagersitz und Implantatteil durch die Krümmung dieser korrespondierend gestalteten Flächen ermöglicht wird. Die Beweglichkeit zwischen den die Lücke begrenzenden Wirbelkörpern lässt sich im Prinzip dabei durch einen Lagersitz zusammenwirkend mit einer Lagerfläche erzielen; im Rahmen der Erfindung ganz besonders bevorzugt ist es aber, wenn die Lagerplatte zweifach vorgesehen ist zur Befestigung einer der Lagerplatten an jedem der Wirbelkörper, und wenn jedes der Implantatteile eine korrespondierend zu dem zugeordneten Lagersitz gekrümmte Lagerfläche aufweist. Durch diese Gestaltung wird eine Beweglichkeit jeweils an den Rändern der Lücke erzielt und vermieden, dass in dem einen Wirbelkörper bzw. dem angrenzenden Implantatteil große Biegebelastungen auftreten.

Weiterhin ist im Rahmen der Erfindung die Gestaltung so gewählt, dass der Lagersitz konkav und die Lagerfläche konvex gekrümmt ist, um so durch die beiden Implantatteile mit den beiden Lagersitzen eine Art Kugelgelenk zu realisieren, das eine Beweglichkeit nicht nur um eine vorgegebene Drehachse ermöglicht, sondern neben Kippbewegungen auch Drehbewegungen der Wirbelsäule zulässt.

Wenn der Krümmungsradius der Lagerfläche der beiden Implantatteile gleich ist, dann wird durch diese Gestaltung eine Übersetzung zwischen den beiden Lagersitzen und damit eine erhöhte Krafteinleitung in einen der Lagersitze vermieden.

Um einen sicheren Sitz der Lagerschale an den Wirbelkörper zu erzielen, weist die Lagerschale auf der am Wirbelkörper zur Anlage kommenden Seite mindestens einen Rastzahn auf, wobei es bevorzugt ist, wenn der Rastzahn eine Schneide besitzt und mehrfach vorgesehen ist. Dabei bietet es sich an, dass die Rastzähne auf einem Kreis angeordnet sind, um so auf den Umfang des Kreises eine gleichmäßige Krafteinleitung in den Wirbelkörper zu erzielen. Weiterhin für eine sichere Befestigung der Lagerschale an dem Wirbelkörper dient, dass die Lagerplatte auf der vom Lagersitz weg weisenden Seite zum lateralen Übergreifen des Wirbelkörpers einen Plattensteg aufweist. Dieser Plattensteg definiert nicht nur die seitliche Ausrichtung der Lagerplatte gegenüber dem Wirbelkörper, sondern bietet darüber hinaus auch die Möglichkeit, dass mindestens eine Knochenschraube vorgesehen ist zur Befestigung des mindestens eine Öffnung aufweisenden Plattenstegs an den Wirbelkörper.

Durch die erfindungsgemäße Gestaltung, bei der die Relativbewegung der Implantatteile gegenüber dem Wirbelkörper über die Lagerplatten vermittelt wird, ergibt sich die Möglichkeit, dass die beiden Implantatteile hülsenartig gestaltet und über ein Innengewinde und ein Außengewinde miteinander verbunden sind, da so zwar bei der Distraktion der beiden Implantatteile eine Relativdrehung dieser Implantatteile gegenüber der Lagerplatte erfolgt, diese Relativdrehung aber unkritisch ist, weil nicht unmittelbar ein Drehmoment auf die Wirbelkörper ausgeübt wird, sondern lediglich die konvex gekrümmte Lagerfläche über den konkav geformten Lagersitz gleitet. Um allerdings bei der Distraktion der beiden Implantatteile diese Gleitbewegung zu vermeiden, kann in bewährter Weise die Gestaltung so gewählt werden, dass die beiden Implantatteile als Hülse gestaltet sind, die axial ineinander greifen, axial gegeneinander verschieblich geführt und gegen gegenseitiges Verdrehen um die Hülsenachse gesichert sind, wobei ein ein Innengewinde aufweisendes mittleres Implantat vorgesehen ist, und wobei eine der Hülse mit dem mittleren Implantatteil gewindemäßig verbunden ist und sowohl vom mittleren Implantatteils auch von der anderen Hülse übergriffen wird. Bei dieser Ausführungsform wird die zur Distraktion des Implantats erforderliche Drehbewegung von dem mittleren Implantatteil ausgeübt, während die beiden Implantatteile gegen Verdrehen gesichert sind und gegenüber dem mittleren Implantatteil nach Art einer Spindel verstellt werden.

Vorteilhaft ist es weiterhin, wenn die Hülsen Aussparungen aufweisen, um so das Einsprossen von Gewebe in das Hülseninnere zu ermöglichen bzw. aktiv Knochenspänne oder Knochenzement einführen zu können.

Vorgesehen ist weiterhin, dass das mittlere Implantatteil gleichmäßig über den Umfang verteilt angeordnete Schlüsselöffnungen aufweist, die genutzt werden können, um durch den Operateur ein Werkzeug einzuführen und die Verdrehung des mittleren Implantatteils zu bewirken. Um nach der erzielten Distraktion des Implantats auf die erforderliche Länge diese Länge auch dauerhaft bereitstellen und sichern zu können, weist eine der Hülsen im Überlappungsbereich mit der anderen Hülse eine Gewindebohrung zur Aufnahme einer Sicherungsschraube auf.

Als günstig hat es sich erwiesen, wenn die Implantatteile und/oder die Lagerplatten aus Titan oder Stahl oder PEEK gebildet sind, wobei die Wahl von Titan oder PEEK insbesondere die Möglichkeit eröffnet, bildgebende Verfahren mittels der Kernspindelresonanztomographie ohne störende Artefakte auszuführen.

Vorteile bei der Anpassung an die Größe der Wirbel bietet es, wenn die Lagerplatte in ihrer Längenausdehnung veränderbar und fixierbar ist, insbesondere wenn die Lagerplatte ausserhalb des Lagersitzes geteilt gestaltet ist mit gegeneinander verstellbaren Bauteilen. Die einfache Veränderung der Längenausdehnung ist dabei durch einen teleskopartigen Aufbau oder eine in einem Gewinde spindelnde Geweindestange erzielbar, deren Lage durch eine Klemmschraube fixiert ist.

Im folgenden wird die Erfindung an einem in der Zeichnung dargestellten Ausführungsbeispiel näher erläutert; es zeigen:

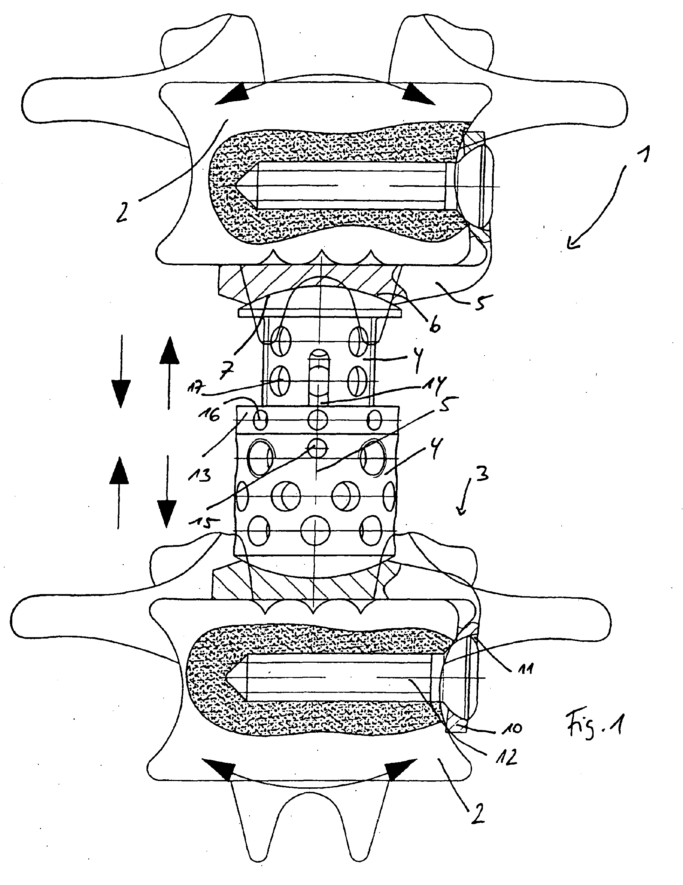

- Fig. 1: eine Seitenansicht eines in die durch das Entfernen eines Wirbelkörpers entstandene Lücke zwischen zwei verbliebenen Wirbelkörpern eingesetzten Implantats,

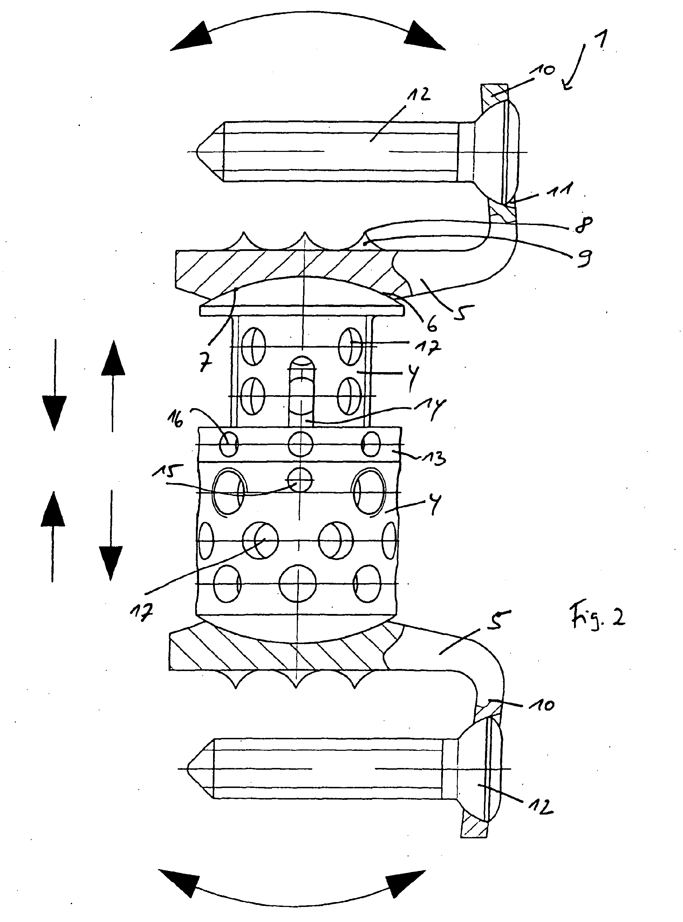

- Fig. 2: eine der Figur 1 entsprechende Darstellung ohne die zur besseren Übersicht in Figur 1 dargestellten Wirbelkörper,
- Fig. 3: eine der Figur 2 entsprechende Darstellung mit einer aus der Neutralstellung erfolgten Auslenkung, und
- Fig. 4: eine der Figur 2 entsprechende Darstellung mit einer gegenüber Figur 3 in die andere Richtung erfolgten Auslenkung.

In der Zeichnung ist ein Implantat 1 dargestellt, das zum Einsetzen zwischen zwei Wirbelkörper 2 der Wirbelsäule dient als Platzhalter für aus der Wirbelsäule entfernte Bandscheiben und Wirbel oder Wirbelteile, wobei dieses erfindungsgemäße Implantat 1 auch dann Verwendung finden kann, wenn ein Wirbelkörper 2 nicht vollständig aus der Wirbelsäule entfernt worden ist, sondern beispielsweise die Kleinwirbel-Gelenke 3 zum Verbleiben in der Wirbelsäule gerettet werden konnten. Dieses Implantat 1 umfasst zwei Implantatteile 4, die in Richtung ihrer koaxialen Längsachse 5 zur Längenänderung des Implantats 1 gegeneinander verstellbar sind, also durch die Längenänderung eine Distraktionsmöglichkeit während der Operation geschaffen ist, um die Länge des Implantats 1 auf die individuellen Erfordernisse einstellen zu können.

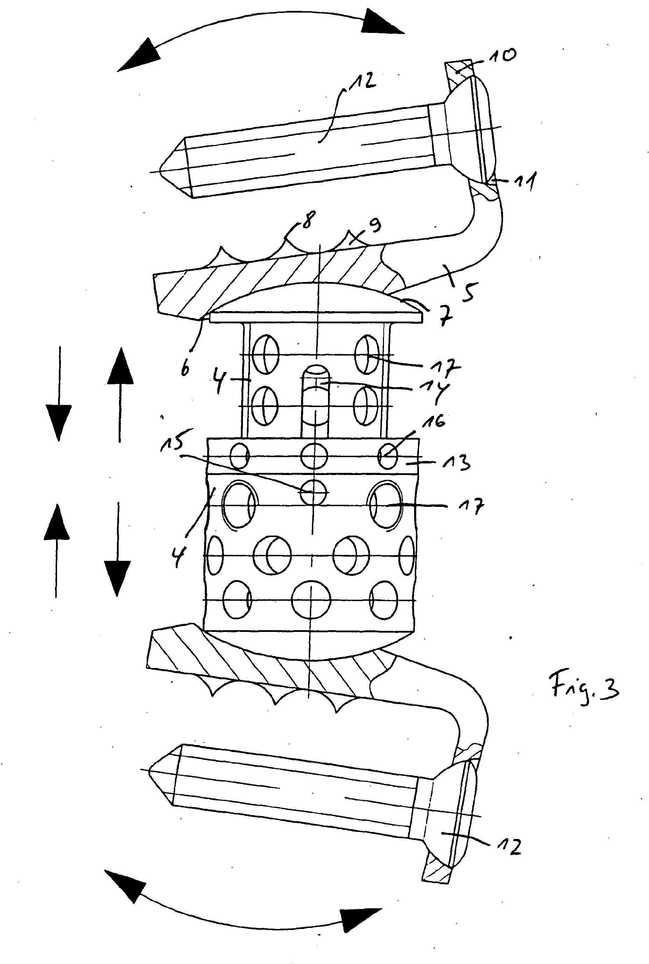

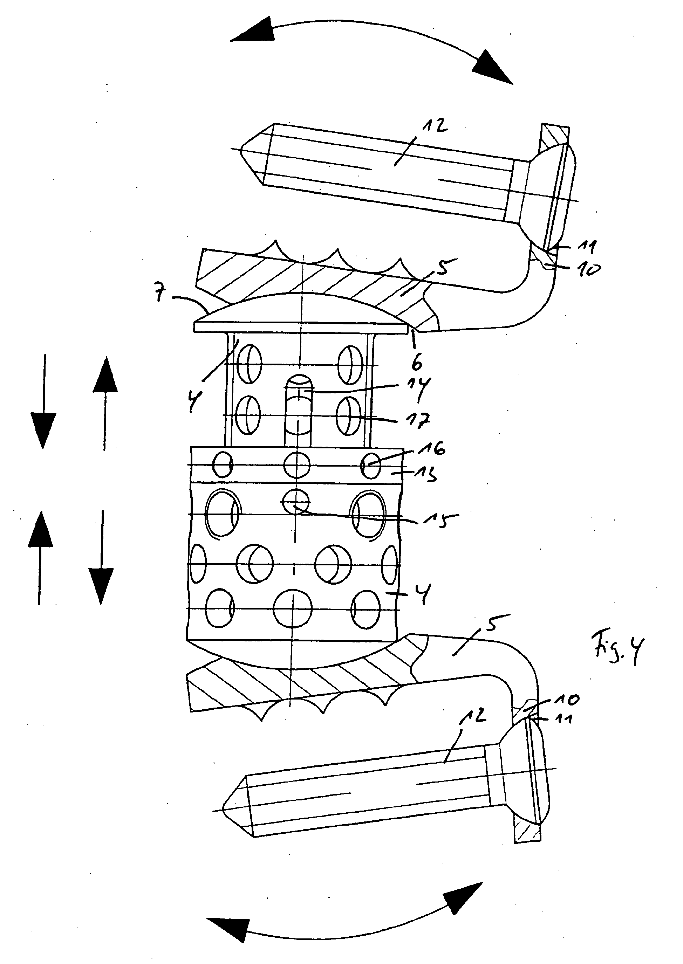

Die Zeichnung lässt erkennen, dass das Implantat 1 eine an dem oberen Wirbelkörper 2 befestigbare Lagerplatte 5 mit einem gekrümmten Lagersitz 6 aufweist, an dem das in der Zeichnung obere Implantatteil 4 mit einer korrespondierend gekrümmten Lagerfläche 7 anliegt. Vergleichbar ist die Abstützung des Implantats 1 gegenüber dem in der Zeichnung unteren Wirbelkörper 2 realisiert, so dass die Lagerplatte 5 insgesamt zweifach vorgesehen ist zur Befestigung einer der Lagerplatten 5 in jedem der Wirbelkörper 2. Dabei sind die Lagersitze 6 konkav und die Lagerflächen 7 konvex gekrümmt mit einem übereinstimmenden Krümmungsradius der Lagerflächen 7 der beiden Implantatteile 4. Diese beiden Aussparungen 17 aufweisenden Implantatteile 4 realisieren damit ein Drehgelenk mit einem zwischen den beiden Lagerflächen 7 liegenden Drehpunkt, wobei aufgrund der geeigneten Gestaltung die Drehachse selber nicht fest vorgegeben ist, so dass das erfindungsgemäße Implantat Drehungen in verschiedenen Richtungen zulässt. Bemerkenswert ist dabei, wie in den Figuren 3 und 4 angedeutet, dass die Drehung des oberen Wirbelkörpers 4 und des unteren Wirbelkörpers 4 dabei nicht gleichsinnig erfolgen müssen, um so den natürlichen Bewegungsablauf bei einer Krümmung der Wirbelsäule zu ermöglichen.

Der Zeichnung ist weiterhin zu entnehmen, dass die Lagerplatte 5 auf der am Wirbelkörper 2 zur Anlage kommenden Seite eine Mehrzahl eine Schneide 8 aufweisende Rastzähne 9 besitzt, die auf dem Umfang eines Kreises angeordnet sind. Diese Rastzähne 9 dienen der sicheren Verankerung der Lagerplatte 5 an dem Wirbelkörper 2 und vermeiden insbesondere eine gleitende Verschiebung der Lagerplatte 5 gegenüber der Wirbelkörperoberfläche. Zur Sicherung der Lagerplatte 5 gegenüber dem Wirbelkörper 2 ist weiterhin auf der vom Lagersitz 6 wegweisenden Seite zum lateralen Übergreifen des Wirbelkörpers 2 ein Plattensteg 10 ausgebildet, der eine Öffnung 11 aufweist, die von einer Knochenschraube 12 durchsetzt wird, die in den Wirbelkörper 2 eingreift.

Die Zeichnung läßt weiterhin erkennen, dass die beiden Implantatteile 4 hülsenartig gestaltet und über ein Innengewinde und ein Außengewinde miteinander verbunden sind, wobei als Träger des Innengewindes ein mittleres Implantatteil 13 genutzt ist, dass mit einer der Hülsen gewindemäßig verbunden ist. Dabei greifen die beiden Implantatteile 4 axial ineinander und sind axial gegeneinander verschieblich geführt und gegen gegenseitiges Verdrehen um die Hülsenachse gesichert, wobei diese Sicherung in einfacher Weise erzielt wird, indem in einem Längsschlitz 14 einen Stift 15 eingreift. Zur Längenverstellung des Implantats 1 wird also das mittlere Implantatteil 13 mittels über den Umfang verteilt angeordneter Schlüsselöffnungen 16 verdreht, so dass die gewindemäßig mit dem mittleren Implantatteil 13 verbundene Hülse in dem mittleren Implantatteil 13 spindel und sich gegenüber dem anderen Implantatteil 4 verstellt. Nach Erreichen der gewünschten Distraktion wird durch eine Sicherungsschraube die relative axiale Lage des einen Implantatteils 4 gegenüber dem anderen Implantatteil 4 festgelegt, indem die Sicherungsschraube durch eine im Überlappungsbereich der einen Hülse mit der anderen Hülse platzierte Gewindebohrung geführt wird.

## Patentansprüche

1. Implantat zum Einsetzen zwischen zwei Wirbelkörper (2) der Wirbelsäule als Platzhalter für aus der Wirbelsäule entfernte Bandscheiben und Wirbel oder Wirbelteile, mit zwei Implantatteilen (4), die in Richtung ihrer koaxialen Langsachsen (5) zur Längenänderung des Implantats (1) gegeneinander verstellbar sind, wobei eine an einem der Wirbelkörper (2) befestigbare Lagerplatte (5) mit einem gekrümmten Lagersitz (6) vorgesehen ist, an dem eines der Implantatteil (4) mit einer korrespondierend gekrümmten Lagerfläche (7) anliegt, wobei die Lagerplatte (5) auf der am Wirbelkörper (2) zur Anlage kommenden Seite mindestens einen Rastzahn (9) aufweist, wobei der Rastzahn (9) eine Schneide (8) besitzt und mehrfach vorgesehen ist, **dadurch gekennzeichnet, dass** die Rastzähne (9) auf einem Kreis angeordnet sind, dass die Lagerplatte (5) auf der vom Lagersitz (6) weg weisenden Seite zum lateralen Übergreifen des Wirbelkörpers (2) einen Plattensteg (10) aufweist und dass mindestens eine Knochenschraube (12) vorgesehen ist zur Befestigung des mindestens eine Öffnung (11) aufweisenden Plattenstegs (10) an dem Wirbelkörper (2).

2. Implantat nach Anspruch 1, **dadurch gekennzeichnet, dass** die Lagerplatte (5) zweifach vorgesehen ist zur Befestigung einer der Lagerplatten (5) an jedem der Wirbelkörper (2), und dass jedes der Implantatteile (4) eine korrespondierend zu dem zugeordneten Lagersitz (6) gekrümmte Lagerfläche (7) aufweist.

3. Implantat nach Anspruch 1 oder 2, **dadurch gekennzeichnet, dass** der Lagersitz (6) konkav und die Lagerfläche (7) konvex gekrümmt ist.

4. Implantat nach Anspruch 2 oder 3, **dadurch gekennzeichnet, dass** der Krümmungsradius der Lagerfläche (7) der beiden Implantatteile (4) gleich ist.

5. Implantat nach einem der Ansprüche 1 bis 4, **dadurch gekennzeichnet, dass** die beiden Implantatteile (4) hülsenartig gestaltet und über ein Innengewinde und ein Aussengewinde miteinander verbunden sind.

6. Implantat nach einem der Ansprüche 1 bis 4, **dadurch gekennzeichnet, dass** die beiden Implantatteile (4) als Hülsen gestaltet sind, die axial ineinander greifen, axial gegeneinander verschieblich geführt und gegen gegenseitiges Verdrehen um die Hülsenachse gesichert sind, dass ein ein Innengewinde aufweisendes mittleres Implantatteil (13) vorgesehen ist, und dass eine der Hülsen mit dem mittleren Implantatteil (13) gewindemässig verbunden ist und sowohl vom mittleren Implantatteil (13) als auch von der anderen Hülse übergriffen wird.

7. Implantat nach Anspruch 5 oder 6, **dadurch gekennzeichnet, dass** die Hülsen Aussparungen (17) aufweisen.

8. Implantat nach Anspruch 6 oder 7, **dadurch gekennzeichnet, dass** das mittlere Implantatteil (13) gleichmässig über den Umfang verteilt angeordnete Schlüsselöffnungen (16) aufweist.

9. Implantat nach einem der Ansprüche 6 bis 8, **dadurch gekennzeichnet, dass** eine der Hülse im Überlappungsbereich mit der anderen Hülse eine Gewindebohrung zur Aufnahme einer Sicherungsschraube aufweist.

10. Implantat nach einem der Ansprüche 1 bis 9, **dadurch gekennzeichnet, dass** die Implantatteile (4) und/oder die Lagerplatten (5) aus Titan oder Stahl oder PEEK gebildet sind.

11. Implantat nach einem der Ansprüche 1 bis 11, **dadurch gekennzeichnet, dass** die Lagerplatte (5) in ihrer Längenausdehhung veränderbar und fixierbar ist.

12. Implantat nach Anspruch 11, **dadurch gekennzeichnet, dass** die Lagerplatte (5) ausserhalb des Lagersitzes (6) geteilt gestaltet ist mit gegeneinander verstellbaren Bauteilen.

## Claims

1. An implant for insertion between two vertebrae (2) of the spinal column as a place holder for vertebrae or vertebra portions and discs removed from the spinal column, comprising two implant portions (4) which are displaceable relative to each other in the direction of their coaxial longitudinal axes (5) for altering the length of the implant (1), wherein there is provided a mounting plate (5) which can be secured to one of the vertebrae (2) and has a curved mounting seat (6) against which one of the implant portions (4) bears with a correspondingly curved mounting surface (7), wherein the mounting plate (5) has at least one latching tooth (9) at the side coming to bear against the vertebra (2), wherein the latching tooth (9) has a cutting edge (8) and is provided in a multiple array, **characterised in that** the latching teeth (9) are arranged on a circle, the mounting plate (5) has a plate leg (10) on the side facing away from the mounting seat (6) for laterally engaging over the vertebra (2) and there is provided at least one bone screw (12) for securing the plate leg (10) which has at least one opening (11) to the vertebra (2).

2. An implant according to claim 1 **characterised in that** there are provided two mounting plates (15) for fixing one of the mounting plates (5) to each of the vertebrae (2) and each of the implant portions (4) has a mounting surface (7) curved corresponding to the associated mounting seat (6).

3. An implant according to claim 1 or claim 2 **characterised in that** the mounting seat (6) is concavely curved and the mounting surface (7) is convexly curved.

4. An implant according to claim 2 or claim 3 **characterised in that** the radius of curvature of the bearing surface (7) of the two implant portions (4) is the same.

5. An implant according to one of claims 1 to 4 **characterised in that** the two implant portions are of a sleeve-like configuration and are connected together by way of a female screwthread and a male screwthread.

6. An implant according to one of claims 1 to 4 **characterised in that** the two implant portions (4) are in the form of sleeves which engage axially into each other, which are guided displaceably axially relative to each other and are secured against rotation relative to each other about the sleeve axis, there is provided a central implant portion (13) having a female screwthread, and one of the sleeves is threadedly connected to the central implant portion (13) and has both the central implant portion (13) and also the other sleeve engaging thereover.

7. An implant according to claim 5 or claim 6 **characterised in that** the sleeves have apertures (17).

8. An implant according to claim 6 or claim 7 **characterised in that** the central implant portion (13) has wrench openings (16) arranged distributed uniformly over the periphery.

9. An implant according to one of claims 6 to 8 **characterised in that** one of the sleeves in the overlap region with the other sleeve has a screwthreaded bore for receiving a securing screw.

10. An implant according to one of claims 1 to 9 **characterised in that** the implant portions (4) and/or the mounting plates (5) are formed from titanium or steel or PEEK.

11. An implant according to one of claims 1 to 11 **characterised in that** the mounting plate (5) is variable and fixable in its longitudinal extent.

12. An implant according to claim 11 **characterised in that** the mounting plate (5) is divided outside the mounting seat (6) with mutually displaceable components.

## Revendications

1. Implant destiné à être inséré en tant qu'élément espaceur entre deux vertèbres (2) de la colonne vertébrale lors d'une ablation de disque vertébral, de vertèbre ou d'une partie de vertèbre, comportant deux éléments d'implant (4) déplaçables l'un par rapport à l'autre dans la direction de leurs axes longitudinaux (5) mutuellement coaxiaux aux fins de faire varier la longueur axiale de l'implant (1), une plaque d'appui (5) à fixer à l'une des vertèbres (2), pourvue d'un siège (6) incurvé sur lequel l'un des éléments d'implant (4) repose par une surface d'appui (7) à courbure adaptée étant prévue, la plaque d'appui (5) comportant sur sa face venant en contact avec la vertèbre (2) au moins un dent d'accrochage (9), la (9) présentant une arête (8) et plusieurs dents d'accrochage étant prévues, **caractérisé en ce que** les dents d'accrochage (9) sont disposées sur un cercle, **en ce que** la plaque d'appui (5), sur sa face éloignée du siège (6), présente une aile de plaque d'appui (10) qui enserre latéralement la vertèbre (2) et **en ce qu'**il est prévu au moins une vis à os (12) pour la fixation à la vertèbre (2) de l'aile de plaque d'appui (10) munie d'au moins une ouverture (11).

2. Implant selon la revendication 1, **caractérisé en ce qu'**il est prévu deux plaques d'appui (5) destinées à être fixées chacune à une vertèbre (2) et **en ce que** chaque élément d'implant (4) comporte une surface d'appui (7) à courbure adaptée au siège (6) concerné.

3. Implant selon la revendication 1 ou 2, **caractérisé en ce que** le siège (6) présente une courbure concave et la surface d'appui (7) une courbure convexe.

4. Implant selon la revendication 2 ou 3, **caractérisé en ce que** le rayon de courbure de la surface d'appui (7) des deux éléments d'implant (4) est identique.

5. Implant selon une des revendications 1 à 4, **caractérisé en ce que** les deux éléments d'implant (4) sont conformés en manchons et sont liés l'un à l'autre par un filetage extérieur et un filetage intérieur.

6. Implant selon une des revendications 1 à 4, **caractérisé en ce que** les deux éléments d'implant (4) sont conformés en manchons qui s'engagent axialement l'un dans l'autre, sont guidés l'un dans l'autre avec possibilité de coulissement et sont immobilisés en rotation l'un par rapport à l'autre autour de l'axe de manchon, **en ce qu'**il est prévu un élément d'implant intermédiaire (13) pourvu d'un filetage intérieur et **en ce que** l'un des manchons est lié par filetage à l'élément d'implant intermédiaire et est entouré à la fois par l'élément d'implant intermédiaire (13) et par l'autre manchon.

7. Implant selon la revendication 5 ou 6, **caractérisé en ce que** les manchons sont pourvus d'évidements (17).

8. Implant selon la revendication 6 ou 7, **caractérisé en ce que** l'élément d'implant intermédiaire (13) présente des ouvertures pour clé (16) réparties régulièrement sur son pourtour.

9. Implant selon une des revendications 6 à 8, **caractérisé en ce que** l'un des manchons, dans la zone de chevauchement avec l'autre manchon, est pourvu d'un trou fileté pour recevoir une vis de blocage.

10. Implant selon une des revendications 1 à 9, **caractérisé en ce que** les éléments d'implant (4) et/ou les plaques d'appui (5) sont réalisées en titane ou en acier ou en PEEK.

11. Implant selon une des revendications 1 à 10, **caractérisé en ce que** la plaque d'appui (5) est modifiable en longueur et peut être bloquée.

12. Implant selon la revendication 11, **caractérisé en ce que** la plaque d'appui (5) en dehors de la région du siège (6) est divisée avec des éléments déplaçables l'un par rapport à l'autre.
